# FASCICULE DE BREVET EUROPEEN

(11) **EP 3 082 880 B1**
(45) Date de publication et mention de la délivrance du brevet: **20.02.2019**
(21) Numéro de dépôt: 14823958.5
(22) Date de dépôt: 16.12.2014
(51) Int. Cl.: A61L 2/07, A23L 3/16

(54) **CUVE DE TRAITEMENT THERMIQUE D'UN LIQUIDE CONTAMINÉ PAR DES AGENTS PATHOGÈNES, INSTALLATION DE DÉCONTAMINATION COMPRENANT UNE TELLE CUVE ET PROCÉDÉ DE DÉCONTAMINATION ASSOCIÉ**
WÄRMEBEHANDLUNGSBEHÄLTER FÜR EINE MIT PATHOGENEN ERREGERN KONTAMINIERTE FLÜSSIGKEIT, DEKONTAMINATIONSEINRICHTUNG MIT EINEM DERARTIGEN BEHÄLTER UND ZUGEHÖRIGES DEKONTAMINIERUNGSVERFAHREN
THERMAL-TREATMENT VESSEL FOR A LIQUID CONTAMINATED BY PATHOGENIC AGENTS, DECONTAMINATION FACILITY COMPRISING SUCH A VESSEL AND ASSOCIATED DECONTAMINATION METHOD

(30) Priorité: 20.12.2013 FR 1363330
(43) Date de publication de la demande: 26.10.2016
(73) Titulaire: Actini, 74500 Maxilly Sur Leman (FR)
(72) Inventeur: DE STOUTZ, Frédéric Laurent, F-74500 Larringes (FR)
(74) Mandataire: Croonenbroek, Thomas Jakob
(86) Numéro de dépôt international: PCT/EP2014/078067
(87) Numéro de publication internationale: WO 2015/091553

(56) Documents cités:
- WO-A1-2006/032701
- WO-A1-2013/133736
- US-A- 2 909 985
- US-A- 3 139 812

## Description

La présente invention concerne une cuve de traitement thermique d'un liquide contaminé par des agents pathogènes, une installation de décontamination comprenant une telle cuve, et un procédé de décontamination associé.

L'invention s'applique notamment dans le cadre des unités de traitement de liquides ou effluents pathogènes contaminés par des germes ou agents infectieux tels que des virus, des bactéries, des parasites(protozoaires, helminthes) ou encore des protéines de type prions ou bien des organismes génétiquement modifiés.

Dans les laboratoires, la fabrication par exemple de vaccins génère des effluents pathogènes qui doivent être décontaminés de façon sûre avant tout rejet par exemple dans le réseau des eaux usées, c'est-à-dire il faut être sûr qu'après traitement, les germes ou agents infectieux ont été rendus inoffensifs.

Ceci peut se faire par exemple en chauffant les effluents dans une cuve hermétique à une température de 135°C et en maintenant cette température pendant quelques minutes.

Or, un des problèmes de ces cuves connu consiste à obtenir une température homogène dans la cuve et d'éviter des zones moins chaudes qui présentent le risque que les germes ou agents ne seront pas complètement neutralisés et rendus inoffensifs.

Un autre problème de ces cuves est d'une part le temps de chauffe qui limite la quantité d'effluent pouvant être traitée en une journée et d'autre part l'énergie nécessaire pour la chauffe et son cout associé.

Le document US 3 139 812 décrit un procédé et un appareil pour cuisiner et stériliser de façon continue des liquides et suspensions. Dans ce document, le liquide est injecté dans une cuve sous forme d'un spray dans un brouillard de vapeur d'eau. Une partie résiduelle liquide au niveau du fond n'est soumise à aucun chauffage.

Il est à noter que ces procédé et appareil ne conviennent aucunement à la décontamination de liquides ou effluents pathogènes contaminés par des germes ou agents infectieux tels que des virus, des bactéries, des parasites (protozoaires, helminthes) ou encore des protéines de type prions ou bien des organismes génétiquement modifiés. En effet, on ne maîtrise pas le temps d'exposition du spray à la vapeur d'eau ni la durée de chauffe à une température élevée.

Par ailleurs, dans un mode de réalisation, le fond de la cuve est équipé de plaques verticales pour éviter tout mouvement de tournoiement du liquide résiduel pour faciliter l'évacuation du liquide par une conduite centrale disposée dans le fond de la cuve.

Les documents WO2013/133736 A1, WO2006/032701 A1 et US2,909,985 sont aussi connus dans le domaine de décontamination.

Afin de pallier au moins partiellement les défauts précédemment mentionnés, l'invention a pour objet une cuve de traitement thermique améliorée.

A cet effet, l'invention a pour objet une cuve de traitement thermique de liquides pathogènes par lots destinée à être remplie à un niveau prédéterminé en laissant un dôme de compression, caractérisée en ce qu'elle comprend au moins un injecteur de vapeur d'eau sous pression dirigé vers l'intérieur de la cuve de traitement thermique avec au moins une composante parallèle à l'axe de révolution de la cuve de traitement thermique et disposé de manière à plonger dans le liquide contaminé par des agents pathogènes lorsque la cuve de traitement thermique est remplie au niveau prédéterminé avec le liquide contaminé par des agents pathogènes et dirigé vers l'intérieur de la cuve de traitement thermique avec au moins une composante tangentielle de manière à chauffer le liquide à décontaminer et à créer un mouvement cyclonique du liquide contaminé par des agents pathogènes à décontaminer afin de permettre d'homogénéiser la température du liquide à décontaminer sans élément mécanique mobile.

Ainsi, en injectant de la vapeur d'eau sous pression, on chauffe le liquide contaminé par des agents pathogènes et on crée un effet cyclonique lors de l'injection de la vapeur d'eau.

Ceci a pour effet d'homogénéiser la répartition de température à l'intérieur de la cuve de traitement et de réduire le temps de chauffe. De plus aucun élément mécanique mobile n'est nécessaire pour mettre le liquide en mouvement, ce qui réduit les coûts de revient d'une telle cuve de traitement thermique et ses coûts de maintenance.

La cuve de traitement thermique selon l'invention peut en outre présenter une ou plusieurs des caractéristiques suivantes prises seules ou en combinaison:
- Ledit au moins un injecteur est dirigé perpendiculaire à une direction radiale vis-à-vis de l'axe de révolution de la cuve de traitement thermique.
- L'injecteur est dirigé vers le haut, incluant notamment un angle compris entre 60° et 80°, notamment de 70° avec l'axe de révolution de la cuve.
- La cuve comporte une canne de support traversant la paroi cylindrique de la cuve de traitement thermique et portant ledit au moins un injecteur.

- La cuve comprend un amortisseur de vibration disposé entre la paroi cylindrique de la cuve de traitement thermique et la canne.
- La cuve comprend des capteurs de niveau de remplissage disposés sur la paroi cylindrique de la cuve de traitement thermique.

L'invention concerne également une installation de décontamination comprenant:
- une cuve de stockage du liquide contaminé par des agents pathogènes,
- une cuve de traitement thermique telle que décrite précédemment dont l'entrée est raccordée fluidiquement à la sortie de la cuve de stockage, et
- un échangeur thermique dont l'entrée est raccordée fluidiquement à la sortie de la cuve de traitement thermique.

L'installation de décontamination peut en outre présenter une ou plusieurs des caractéristiques suivantes prises seules ou en combinaison:
- La cuve de stockage est disposée en hauteur par rapport à la cuve de traitement thermique, et la cuve de traitement thermique est disposée en hauteur par rapport à l'échangeur thermique de sorte que la circulation des liquides pathogènes dans l'installation est réalisée par gravité.
- L'installation de décontamination comprend une conduite de délestage avec une vanne de sécurité dont l'entrée est raccordée au niveau du dôme de la cuve de traitement thermique et dont la sortie est raccordée à la cuve de stockage.
- La sortie de la conduite de délestage est située dans la moitié inférieure de la cuve de stockage.

L'invention concerne aussi un procédé de décontamination de liquides pathogènes dans une cuve de traitement thermique telle que décrite précédemment :
- on remplit la cuve de traitement thermique (17) avec un liquide contaminé par des agents pathogènes en laissant un volume gazeux d'expansion à l'intérieur de la cuve de traitement (17),
- on chauffe le liquide contaminé par des agents pathogènes à une température comprise entre 130°C et 140°C, de préférence à 135°C pendant une durée comprise entre 4 min et 6min, de préférence pendant 5min, en injectant de la vapeur d'eau sous pression,
- on évacue le liquide ainsi décontaminé.

D'autres caractéristiques et avantages apparaîtront à la lecture de la description des figures suivante, parmi lesquelles :
- la figure 1 est un schéma de principe de l'installation de décontamination selon un mode de réalisation,
- la figure 2 est une vue en perspective d'une cuve de traitement selon l'invention,
- la figure 3 une vue schématique en coupe transversale de la cuve de la figure 2, au niveau du milieu de la cuve de traitement,
- la figure 4 est un organigramme montrant un exemple de différentes étapes d'un procédé de décontamination, et
- les figures 5 et 6 montrent l'installation de la figure 1 à différentes étapes du procédé de décontamination.

Sur toutes les figures, les mêmes éléments portent les mêmes numéros de référence.

On va décrire ci-après en référence aux différentes figures un mode de réalisation de la présente invention.

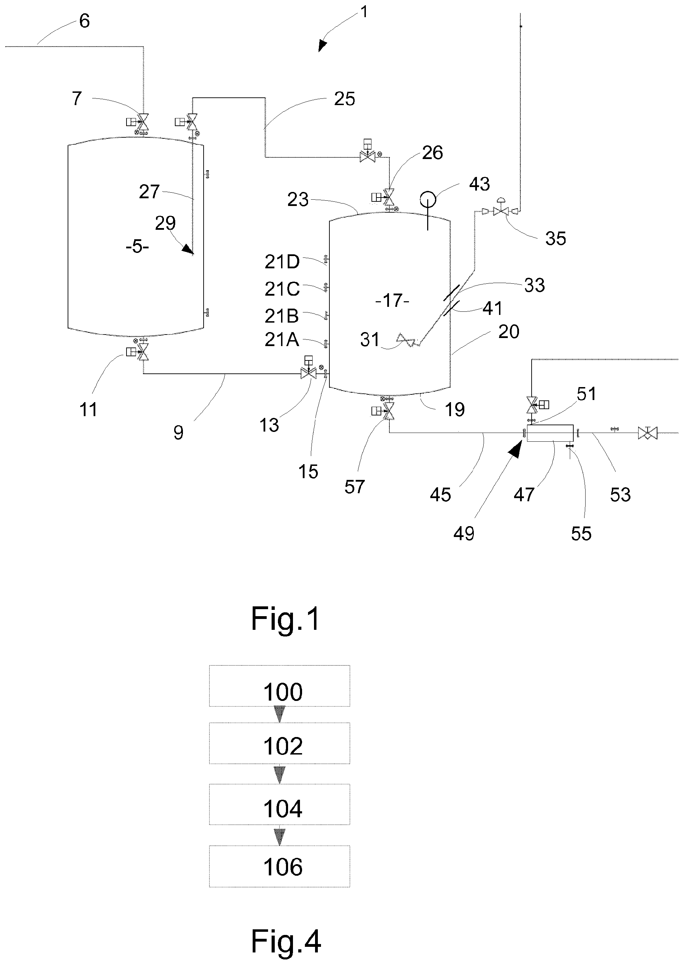

La figure 1 montre un schéma de principe simplifié d'une installation de décontamination 1 selon un mode de réalisation.

Ainsi, l'installation de décontamination 1 comprend une cuve 5 de stockage d'un liquide contaminé par des agents pathogènes. Cette cuve 5, par exemple en acier inoxydable 316 L, est raccordée à une ligne d'entrée 6 et sert à recueillir les liquides pathogènes issus par exemple d'une ligne de production tel qu'une ligne de production de vaccins. Une vanne de commande 7 est disposée à l'entrée de la cuve de stockage 5.

Cette cuve 5 de stockage sert en quelque sorte de tampon avant le traitement des liquides pathogènes par lot (« batch » en anglais).

La sortie de la cuve de stockage 5 est raccordée via une conduite 9 à une entrée 15 d'une cuve de traitement thermique 17, par exemple d'un volume de 3001 en acier inoxydable 316 L. Dans la conduite 9 sont disposées deux vannes 11 et 13.

Cette entrée 15 est disposée dans la partie basse de la cuve de traitement thermique 17 près du fond 19, de sorte que la cuve 17 est remplie par le bas.

Comme cela est représenté sur la figure 1, la cuve de stockage 5 est disposée en hauteur par rapport à la cuve de traitement thermique 17 de sorte que le remplissage de la cuve de traitement 17 depuis la cuve de stockage 5 peut se faire par gravité, selon le principe des vases communicants. On a donc pas besoin d'un autre moyen mécanique comme une pompe pour réaliser le transfert de liquide entre les deux cuves 5 et 17.

De préférence, le volume de la cuve 5 et sa hauteur par rapport à la cuve 17 sont choisis de manière que lorsque la cuve de stockage 5 se vide dans la cuve de traitement thermique 17 selon le principe des vases communicants, il reste un fond de liquide contaminé dans la cuve de stockage 5 et que la cuve 17 de traitement thermique est remplie à une hauteur laissant un dôme gazeux de compression.

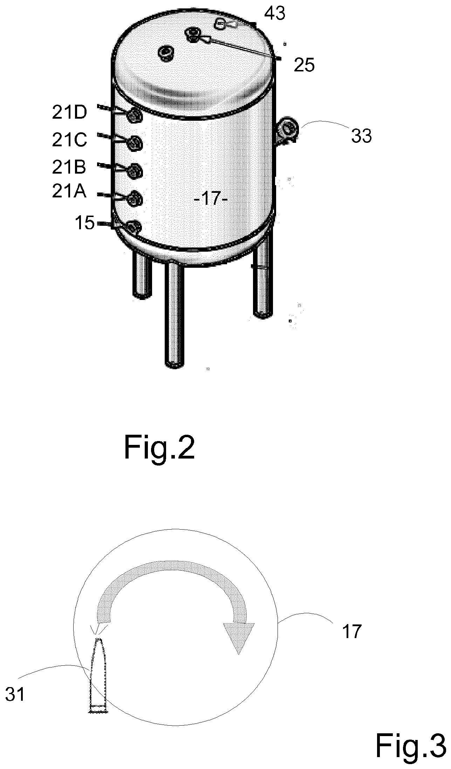

Comme on le voit sur les figures 1 et 2, la cuve de traitement thermique 17 est équipée sur sa paroi cylindrique 20 de quatre sondes 21A, 21B, 21C et 21D de détection du niveau de remplissage.

Comme on le verra plus loin, pour une session de traitement, la cuve de traitement thermique sera remplie à un niveau se situant à la sonde 21C.

En outre, l'installation comprend une conduite de délestage de l'air 25 avec une vanne de sécurité 26 dont l'entrée est raccordée au sommet 23 de la cuve de traitement thermique 17, donc au niveau du dôme de la cuve de traitement thermique 17, et dont la sortie est raccordée à la cuve de stockage 5.

Par conséquent, en cas de surpression dans la cuve de traitement thermique 17, le délestage peut se faire directement dans la cuve de stockage 5 de sorte qu'il n'y a pas de risque de contamination. De plus, on évite ainsi des évents couteux avec des filtres nécessitant des inspections de maintenance régulières.

Comme on le voit sur la figure 1, la conduite de délestage 25 est raccordée au sommet de la cuve de stockage 5 et se prolonge par une canne 27 sensiblement parallèle à l'axe de la cuve 5 et faisant saillie dans cette cuve 5 de sorte que la sortie 29 de la conduite de délestage 25 plonge dans le liquide contaminé à l'état rempli de cette cuve 5. La longueur de la canne 27 est choisie de manière que la sortie 29 se trouve en dessous du niveau du liquide de cette cuve de stockage 5.

De plus, la cuve de traitement thermique 17 comprend au moins un injecteur 31 de vapeur d'eau sous pression, par exemple de 5 bars (voir figures 1 et 3).

Cet injecteur 31 disposé de manière à plonger dans le liquide contaminé par des agents pathogènes lorsque la cuve est remplie au niveau prédéterminé, c'est-à-dire à la sonde 21C (à peu près ¾ du volume total de la cuve de traitement thermique 17) et dirigé vers l'intérieur de la cuve 17 avec au moins une composante tangentielle, voir complètement tangentiellement par rapport à la paroi cylindrique de la cuve 17 de manière à créer un mouvement cyclonique du liquide à décontaminer lorsque de la vapeur d'eau sous pression est injectée via l'injecteur 31.

A cet effet, l'injecteur 31 est porté par une canne de support 33 dont une extrémité est reliée via une vanne de commande 35 à une conduite de vapeur d'eau sous pression. Cette canne de support 33 traverse la paroi 20 de la cuve 17 et fait saillie en étant dirigée vers le fond 19 et porte à son extrémité libre l'injecteur 31.

Par composante tangentielle, il faut comprendre une composante qui est perpendiculaire à une direction radiale par rapport à l'axe de révolution de la cuve 17 de traitement thermique.

De plus, selon l'exemple des figures, l'injecteur 31 est dirigé vers l'intérieur de la cuve 17 avec au moins une composante parallèle à l'axe de révolution cylindrique de la cuve 17, plus spécifiquement l'injecteur 31 est dirigé vers le haut, incluant notamment un angle α compris entre 60° et 80°, notamment de 70° avec l'axe cylindrique de la cuve.

Selon une variante non représentée, l'injecteur 31 peut être dirigé vers le bas en direction du fond 19 de la cuve 17.

Selon encore une autre variante, on peut prévoir plusieurs injecteurs, soit montés sur une seule canne de support, soit chaque injecteur est monté sur une canne de support individuelle.

Afin de limiter des vibrations et la génération de bruit lors de l'injection de la vapeur d'eau, un amortisseur de vibrations 41 est disposé entre la paroi cylindrique 20 de la cuve 17 et la canne 33 de support.

Au sommet 23 de la cuve 17 est en outre installée une sonde de température 43. Cette sonde 43 est reliée à la vanne de régulation 35 pour contrôler par exemple l'injection de la vapeur d'eau sous pression en fonction de la température.

Une sortie au niveau du fond 19 de la cuve de traitement thermique 17 est raccordée via une canalisation 45 à une entrée d'un échangeur thermique 47 destiné à refroidir le liquide décontaminé en sortie de la cuve de traitement thermique 17.

L'échangeur thermique 47 dispose de deux entrées, une entrée de fluide décontaminé 49, une entrée d'eau de refroidissement 51 et de deux sorties, une sortie de liquide décontaminé et refroidi 53 et une sortie d'eau 55 ayant servi de liquide de refroidissement dans l'échangeur thermique 23. Ainsi le réseau d'eau de refroidissement est toujours séparé des canalisations du liquide décontaminé de sorte qu'il n'y a pas de possibilité d'une contamination retour, ce qui augmente encore la sécurité de l'installation 1 décrite ici.

Comme on le voit sur la figure 1 représenté schématiquement, l'échangeur thermique 47 est de forme allongée pour maximiser les surfaces d'échange et est disposé en dessous de la cuve de traitement thermique 17 de sorte que le liquide décontaminé en sortie peut transiter par gravité sans aucune intervention d'une pompe.

Une vanne de régulation 57 (figure 1) disposée dans la canalisation 45 permet de réguler le flux, par exemple pour évacuer la cuve de traitement thermique 17.

On va maintenant décrire un exemple de fonctionnement de l'installation 1 décrite ci-dessus.

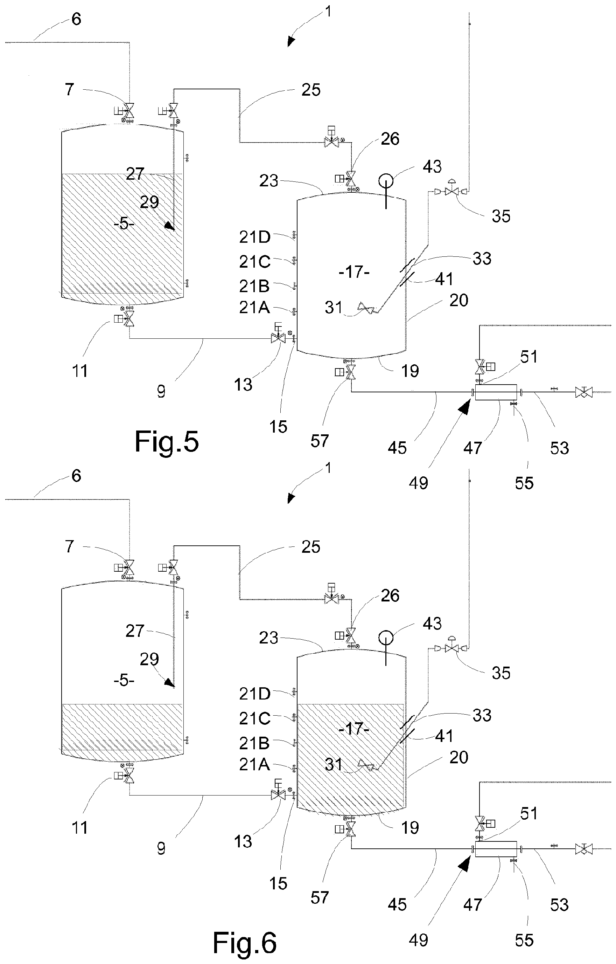

On part de la situation de la figure 5 où la cuve de stockage est remplie à environ 80% d'un liquide contaminé par des agents pathogènes.

Selon une première étape 100 (voir figure 4 et 6), on remplit par le principe des vases communicants la cuve de traitement thermique 17 avec le liquide contaminé jusqu' au niveau se situant à la sonde de niveau 21C et donc en laissant un volume de compression à l'intérieur de la cuve de traitement 17 au niveau du dôme. Pendant cette première étape, le surplus du volume gazeux du dôme de la cuve de traitement thermique 17 est évacué via la conduite de délestage 25 dans la cuve de stockage 5.

Puis, selon une seconde étape 102, on injecte de la vapeur d'eau à une pression de 5bars par exemple dans le liquide contaminé via l'injecteur 31 pour chauffer le liquide contaminé à une température comprise entre 130°C et 140°C, de préférence à 135°C pendant une durée comprise entre 4 min et 6min, de préférence pendant 5min, en laissant augmenter la pression dans la cuve de traitement thermique 17, par exemple ici autour de 4bars. L'injection de la vapeur d'eau par l'injecteur 31 va créer un effet cyclonique qui permet d'homogénéiser la température du liquide à décontaminer. Si la pression dans la cuve de traitement thermique 17 dépasse 4 bars alors que la température est encore inférieure à la température de consigne, le trop plein de pression est évacuée via la conduite de délestage 25 dans la cuve de stockage 5.

Enfin, selon une étape 104, on évacue le liquide ainsi décontaminé à l'aide de la pression qui s'est établie lors du chauffage et de la gravité.

Puis, le liquide décontaminé est refroidi par exemple à environ 60°C lors d'une étape 106 dans l'échangeur thermique 47.

On comprend donc que l'installation de décontamination permet une décontamination efficace et présente une fiabilité accrue. De plus, sa maintenance est aisée et ne présente pas de difficultés particulières.

## Revendications

1. Cuve de traitement thermique (17) de liquides pathogènes par lots destinée à être remplie à un niveau prédéterminé en laissant un dôme de compression, **caractérisée en ce qu'**elle comprend au moins un injecteur (31) de vapeur d'eau sous pression dirigé vers l'intérieur de la cuve de traitement thermique (17) avec au moins une composante parallèle à l'axe de révolution de la cuve de traitement thermique (17) et disposé de manière à plonger dans le liquide contaminé par des agents pathogènes lorsque la cuve de traitement thermique (17) est remplie au niveau prédéterminé avec le liquide contaminé par des agents pathogènes et dirigé vers l'intérieur de la cuve de traitement thermique (17) avec au moins une composante tangentielle de manière à chauffer le liquide à décontaminer et à créer un mouvement cyclonique du liquide contaminé par des agents pathogènes à décontaminer afin de permettre d'homogénéiser la température du liquide à décontaminer sans élément mécanique mobile.

2. Cuve selon la revendication 1, **caractérisée en ce que** ledit au moins un injecteur (31) est dirigé perpendiculairement à une direction radiale vis-à-vis de l'axe de révolution de la cuve de traitement thermique (17).

3. Cuve selon l'une quelconque des revendications 1 ou 2, **caractérisée en ce que** l'injecteur (31) est dirigé vers le haut, incluant notamment un angle compris entre 60° et 80°, notamment de 70° avec l'axe de révolution de la cuve.

4. Cuve selon l'une quelconque des revendications 1 ou 2, **caractérisé en ce que** l'injecteur (31) est dirigé vers le bas en direction du fond (19) de la cuve (17).

5. Cuve selon l'une quelconque des revendications 1 à 4, **caractérisée** ce qu'elle comporte une canne de support (33) traversant la paroi cylindrique (39) de la cuve de traitement thermique (17) et portant ledit au moins un injecteur (31).

6. Cuve selon la revendication 5, **caractérisée en ce qu'**elle comprend un amortisseur de vibration (41) disposé entre la paroi cylindrique (20) de la cuve de traitement thermique (17) et la canne (33).

7. Cuve selon l'une quelconque des revendications 1 à 5, **caractérisée** ce qu'elle comprend des capteurs de niveau de remplissage (21A, 21B, 21C, 21C) disposés sur la paroi cylindrique (20) de la cuve de traitement thermique (17).

8. Installation de décontamination comprenant
- une cuve de stockage du liquide contaminé par des agents pathogènes (5),
- une cuve de traitement thermique (17) selon l'une quelconque des revendications 1 à 6 dont l'entrée (15) est raccordée fluidiquement à la sortie de la cuve de stockage (5), et
- un échangeur thermique (47) dont l'entrée est raccordée fluidiquement à la sortie de la cuve de traitement thermique (17).

9. Installation de décontamination selon la revendication 8, **caractérisée en ce que** la cuve de stockage (5) est disposée en hauteur par rapport à la cuve de traitement thermique (17), et **en ce que** la cuve de traitement thermique (17) est disposée en hauteur par rapport à l'échangeur thermique (47) de sorte que la circulation des liquides pathogènes dans l'installation (1) est réalisée par gravité.

10. Installation de décontamination selon la revendication 8 ou 9, **caractérisée en ce qu'**elle comprend une conduite de délestage (25) avec une vanne de sécurité (26) dont l'entrée est raccordée au niveau du dôme de la cuve de traitement thermique (17) et dont la sortie est raccordée à la cuve de stockage (5).

11. Installation de décontamination selon la revendication 10, **caractérisée en ce que** la sortie (29) de la conduite de délestage (25) est situé dans la moitié inférieure de la cuve de stockage(5).

12. Procédé de décontamination de liquides potentiellement pathogènes dans une cuve de traitement thermique (17) selon l'une quelconque des revendications 1 à 7, **caractérisé en ce que**
- on remplit la cuve de traitement thermique (17) avec un liquide contaminé par des agents pathogènes en laissant un volume gazeux d'expansion à l'intérieur de la cuve de traitement (17),
- on chauffe le liquide contaminé par des agents pathogènes à une température comprise entre 130°C et 140°C, de préférence à 135°C pendant une durée comprise entre 4 min et 6min, de préférence pendant 5min, en injectant de la vapeur d'eau sous pression,
- on évacue le liquide ainsi décontaminé.

13. Procédé de décontamination de liquides potentiellement pathogènes selon la revendication 12, **caractérisé en ce que** l'on laisse augmenter la pression dans la cuve de traitement thermique (17) à une pression de 4bars.

## Patentansprüche

1. Behälter zur thermischen Behandlung (17) pathogener Flüssigkeiten in Chargen, dafür bestimmt, bis auf ein festgelegtes Niveau gefüllt zu werden, wobei ein Druckdom belassen wird, **dadurch gekennzeichnet, dass** er mindestens einen Injektor (31) für Wasserdampf unter Druck umfasst, der mit mindestens einer zur Rotationsachse des Behälters zur thermischen Behandlung (17) parallelen Komponente zum Innenraum des Behälters zur thermischen Behandlung (17) gerichtet und so angeordnet ist, dass er in die durch Krankheitserreger kontaminierte Flüssigkeit eintaucht, wenn der Behälter zur thermischen Behandlung (17) bis zu dem festgelegten Niveau mit der durch Krankheitserreger kontaminierten Flüssigkeit gefüllt ist, und der zum Innenraum des Behälters zur thermischen Behandlung (17) mit mindestens einer tangentialen Komponente gerichtet ist, so dass die zu dekontaminierende Flüssigkeit erhitzt und eine Zyklonbewegung der zu dekontaminierenden durch Krankheitserreger kontaminierten Flüssigkeit erzeugt wird, damit die Temperatur der zu dekontaminierenden Flüssigkeit ohne bewegliches mechanisches Element homogenisiert werden kann.

2. Behälter nach Anspruch 1, **dadurch gekennzeichnet, dass** der mindestens ein Injektor (31) senkrecht zu einer Radialrichtung in Bezug auf die Rotationsachse des Behälters zur thermischen Behandlung (17) gerichtet ist.

3. Behälter nach einem der Ansprüche 1 oder 2, **dadurch gekennzeichnet, dass** der Injektor (31) nach oben gerichtet ist, wobei er insbesondere einen Winkel zwischen 60° und 80°, insbesondere von 70°, mit der Rotationsachse des Behälters einschließt.

4. Behälter nach einem der Ansprüche 1 oder 2, **dadurch gekennzeichnet, dass** der Injektor (31) nach unten in Richtung zum Boden (19) des Behälters (17) gerichtet ist.

5. Behälter nach einem der Ansprüche 1 bis 4, **dadurch gekennzeichnet, dass** er einen Stützstab (33) umfasst, der durch die zylindrische Wand (39) des Behälters zur thermischen Behandlung (17) hindurchgeht und den mindestens einen Injektor (31) trägt.

6. Behälter nach Anspruch 5, **dadurch gekennzeichnet, dass** er einen Schwingungsdämpfer (41) umfasst, der zwischen der zylindrischen Wand (20) des Behälters zur thermischen Behandlung (17) und dem Stab (33) angeordnet ist.

7. Behälter nach einem der Ansprüche 1 bis 5, **dadurch gekennzeichnet, dass** er Füllstandssensoren (21A, 21B, 21C, 21C) umfasst, die an der zylindrischen Wand (20) des Behälters zur thermischen Behandlung (17) angeordnet sind.

8. Dekontaminationsanlage, umfassend
- einen Aufbewahrungsbehälter für die durch Krankheitserreger kontaminierte Flüssigkeit (5),
- einen Behälter zur thermischen Behandlung (17) nach einem der Ansprüche 1 bis 6, dessen Einlass (15) in Flüssigkeitskommunikation mit dem Auslass des Aufbewahrungsbehälters (5) steht, und
- einen Wärmetauscher (47), dessen Einlass in Flüssigkeitskommunikation mit dem Auslass des Behälters zur thermischen Behandlung (17) steht.

9. Dekontaminationsanlage nach Anspruch 8, **dadurch gekennzeichnet, dass** der Aufbewahrungsbehälter (5) oberhalb in Bezug auf den Behälter zur thermischen Behandlung (17) angeordnet ist, und dadurch, dass der Behälter zur thermischen Behandlung (17) oberhalb in Bezug auf den Wärmetauscher (47) angeordnet ist, so dass die Zirkulation der pathogenen Flüssigkeiten in der Anlage (1) mittels Schwerkraft erreicht wird.

10. Dekontaminationsanlage nach Anspruch 8 oder 9, **dadurch gekennzeichnet, dass** sie eine Entlastungsleitung (25) mit einem Sicherheitsventil (26) umfasst, deren Einlass in Höhe des Doms des Behälters zur thermischen Behandlung (17) angeschlossen ist und deren Auslass mit dem Aufbewahrungsbehälter (5) verbunden ist.

11. Dekontaminationsanlage nach Anspruch 10, **dadurch gekennzeichnet, dass** der Auslass (29) der Entlastungsleitung (25) sich in der unteren Hälfte des Aufbewahrungsbehälters (5) befindet.

12. Verfahren zur Dekontamination potenziell pathogener Flüssigkeiten in einem Behälter zur thermischen Behandlung (17) nach einem der Ansprüche 1 bis 7, **dadurch gekennzeichnet, dass**:
- man den Behälter zur thermischen Behandlung (17) mit einer durch Krankheitserreger kontaminierten Flüssigkeit füllt, wobei man ein Gasexpansionsvolumen im Inneren des Behälters zur thermischen Behandlung (17) belässt,
- man die durch Krankheitserreger kontaminierte Flüssigkeit auf eine Temperatur zwischen 130°C und 140°C, vorzugsweise auf 135°C, während einer Dauer zwischen 4 min und 6 min, vorzugsweise während 5 min, erhitzt, wobei Wasserdampf unter Druck eingespritzt wird,
- man die so dekontaminierte Flüssigkeit ablässt.

13. Verfahren zur Dekontamination potenziell pathogener Flüssigkeiten nach Anspruch 12, **dadurch gekennzeichnet, dass** man den Druck in dem Behälter zur thermischen Behandlung (17) auf einen Druck von 4 bar ansteigen lässt.

## Claims

1. Vessel (17) for the heat treatment of pathogenic liquids in batches that is intended to be filled to a predetermined level while leaving a compression dome, **characterized in that** it comprises at least one pressurized steam injector (31) pointed toward the inside of the heat treatment vessel (17) with at least one component parallel to the axis of revolution of the heat treatment vessel (17) and positioned so as to immerse in the liquid contaminated by pathogenic agents when the heat treatment vessel (17) is filled to the predetermined level with the liquid contaminated by pathogenic agents and pointed toward the inside of the heat treatment vessel (17), with at least one tangential component so as to heat the liquid to be decontaminated and to create a cyclonic movement of the liquid contaminated by pathogenic agents to be decontaminated in order to make it possible to homogenize the temperature of the liquid to be decontaminated without a movable mechanical element.

2. Vessel according to Claim 1, **characterized in that** said at least one injector (31) is pointed perpendicular to a radial direction with respect to the axis of revolution of the heat treatment vessel (17).

3. Vessel according to either one of Claims 1 and 2, **characterized in that** the injector (31) is pointed upward, including in particular an angle of between 60° and 80°, in particular of 70° with the axis of revolution of the vessel.

4. Vessel according to either one of Claims 1 and 2, **characterized in that** the injector (31) is pointed downward in the direction of the bottom (19) of the vessel (17).

5. Vessel according to any one of Claims 1 to 4, **characterized in that** it comprises a support tube (33) passing through the cylindrical wall (39) of the heat treatment vessel (17) and bearing said at least one injector (31).

6. Vessel according to Claim 5, **characterized in that** it comprises a vibration damper (41) positioned between the cylindrical wall (20) of the heat treatment vessel (17) and the tube (33).

7. Vessel according to any one of Claims 1 to 5, **characterized in that** it comprises filling level sensors (21A, 21B, 21C, 21C) positioned on the cylindrical wall (20) of the heat treatment vessel (17) .

8. Decontamination facility comprising:
- a vessel (5) for storing the liquid contaminated by pathogenic agents,
- a heat treatment vessel (17) according to any one of Claims 1 to 6, the inlet (15) of which is fluidically coupled to the outlet of the storage vessel (5), and
- a heat exchanger (47), the inlet of which is fluidically coupled to the outlet of the heat treatment vessel (17).

9. Decontamination facility according to Claim 8, **characterized in that** the storage vessel (5) is positioned higher up relative to the heat treatment vessel (17), and **in that** the heat treatment vessel (17) is positioned higher up relative to the heat exchanger (47) so that the circulation of the pathogenic liquids in the facility (1) is achieved by gravity.

10. Decontamination facility according to Claim 8 or 9, **characterized in that** it comprises a relief line (25) with a safety valve (26), the inlet of which is coupled level with the dome of the heat treatment vessel (17), and the outlet of which is coupled to the storage vessel (5).

11. Decontamination facility according to Claim 10, **characterized in that** the outlet (29) of the relief line (25) is located in the lower half of the storage vessel (5).

12. Process for decontaminating potentially pathogenic liquids in a heat treatment vessel (17) according to any one of Claims 1 to 7, **characterized in that**:
- the heat treatment vessel (17) is filled with a liquid contaminated by pathogenic agents while leaving a gaseous expansion volume inside the treatment vessel (17),
- the liquid contaminated by pathogenic agents is heated at a temperature between 130°C and 140°C, preferably at 135°C for a duration of between 4 min and 6 min, preferably for 5 min, by injecting pressurized steam,
- the liquid thus decontaminated is discharged.

13. Process for decontaminating potentially pathogenic liquids according to Claim 12, **characterized in that** the pressure in the heat treatment vessel (17) is allowed to increase to a pressure of around 4 bar.
